(19) Europäisches Patentamt

European Patent Office

Office européen des brevets

(11) Publication number: **0 435 693 B1**

## EUROPEAN PATENT SPECIFICATION

(45) Date of publication of patent specification: **15.03.95**

(51) Int. Cl.⁶: **A61K 31/28**, A61K 31/80,
A61K 31/19, A61K 33/28,
A61K 31/02, A61K 31/405,
A61K 31/71, A61K 31/545,
A61K 31/43, A61K 31/40,
A61K 31/70

(21) Application number: **90314407.9**

(22) Date of filing: **28.12.90**

The file contains technical information submitted after the application was filed and not included in this specification

(54) **Pharmaceutical compositions containing 3-oxygermylpropionic acid polymers for inhibiting the degeneration of cells.**

(30) Priority: **29.12.89 JP 341810/89**
**02.07.90 JP 174961/89**

(43) Date of publication of application:
**03.07.91 Bulletin 91/27**

(45) Publication of the grant of the patent:
**15.03.95 Bulletin 95/11**

(84) Designated Contracting States:
**BE CH DE ES FR GB IT LI SE**

(56) References cited:
**EP-A- 0 186 505**

(73) Proprietor: **SANWA KAGAKU KENKYUSHO CO., LTD.**
**No. 35, Higashi-sotobori-cho**
**Higashi-ku**
**Nagoya-shi**
**Aichi-ken (JP)**

(72) Inventor: **Sawai, Kiichi, c/o Sanwa Kagaku Kenkyusho Co.**
**Ltd.,**
**Higashi-sotobori-cho 35**
**Higashi-ku,**
**Nagoya-shi,**
**Aichi-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 111, no. 5, 31st July 1989, page 32, abstract no. 33298s, Columbus, Ohio, US; K. KUMANO et al.: "The role for reactive oxygen species in cyclosporin A induced nephrotoxicity in rats", & TRANSPLANT. PROC. 1989, 21(1, BOOK 1), 941-2

IDEM

CHEMICAL ABSTRACTS, vol. 111, no. 5, 31st July 1989, page 315, abstract no. 36179q, Columbus, Ohio, US; Y. MASAKI et al.: "Protective effect of an organic germanium compound on warm ischemia and prolonged kidney preservation", & TRANSPLANT. PROC. 1989, 21(1, BOOK 2), 1250-1

CHEMICAL ABSTRACTS, vol. 109, no. 23, 5th December 1988, page 31, abstract no. 204547n, Columbus, Ohio, US; S. FURUSAWA et al.: "Reduction of cis-diamminedichloroplatinum-induced renal toxicity by 2-carboxyethylgermanium sesquioxide", & ANNU. REP. TOHOKU COLL. PHARM. 1987, (34), 269-75

IDEM

CHEMICAL ABSTRACTS, vol. 97, no. 19, 8th November 1982, page 40, abstract no. 156062k, Columbus, Ohio, US; S. ARIMORI et al.: "Effect of Ge-132 on L1210", & INT. CONGR. SER. EXCERPTA MED. 1982, 563(IMMUNOMODULATION MICROB. PROD. RELAT. SYNTH. COMPD.), 536-9

IDEM

CHEMICAL ABSTRACTS, vol. 105, no. 23, 8th December 1986, page 25, abstract no. 202828y, Columbus, Ohio, US; H. KOBAYASHI et al.: "Effect of combination immunochemotherapy with organogermanium compound, GE 132, and antitumor agents on C57BL/6 mice bearing Lewis lung carcinoma (3LL)", & GAN TO KAGAKU RYOHO 1986, 13(8), 2588-93

IDEM

DRUGS OF THE FUTURE, vol. 13, no. 5, 1988, pages 441-453; K. MIYAO et al.: "Carboxyethylgermanium sesquioxide and related organogermanium compounds - unique synthetic BRM's - A review"

Inventor: **Kurono, Masayasu, c/o Sanwa Kagaku Kenkyusho Co. Ltd.,** Higashi-sotobori-cho 35 Higashi-ku, Nagoya-shi, Aichi-ken (JP)

Inventor: **Nakano, Kazumasa, c/o Sanwa Kagaku Kenkyusho Co. Ltd.,** Higashi-sotobori-cho 35 Higashi-ku, Nagoya-shi, Aichi-ken (JP)

Inventor: **Asano, Kyoichi, c/o Sanwa Kagaku Kenkyusho Co. Ltd.,** Higashi-sotobori-cho 35 Higashi-ku, Nagoya-shi, Aichi-ken (JP)

Inventor: **Mitani, Takahiko, c/o Sanwa Kagaku Kenkyusho Co. Ltd.,** Higashi-sotobori-cho 35 Higashi-ku, Nagoya-shi, Aichi-ken (JP)

Inventor: **Ninomiya, Naohisa, c/o Sanwa Kagaku Kenkyusho Co. Ltd.,** Higashi-sotobori-cho 35 Higashi-ku, Nagoya-shi, Aichi-ken (JP)

Inventor: **Kato, Bunkichi, c/o Sanwa Kagaku Kenkyusho Co. Ltd.,** Higashi-sotobori-cho 35 Higashi-ku, Nagoya-shi, Aichi-ken (JP)

(74) Representative: **Moore, Anthony John et al Gee & Co. Chancery House Chancery Lane London WC2A 1OU (GB)**

PATENT ABSTRACTS OF JAPAN, vol. 11, no. 398 (C-466)[2345], 25th December 1987; & JP-A-62 161 724 (SANWA KAGAKU KEN-KYUSHO K.K.) 17-07-1987

**Description**

This invention relates to the use of compositions for the manufacture of a medicament inhibiting the degeneration of liver cells caused by drugs, and relieving the toxicity of drugs to the liver and kidneys, the composition containing as a pharmaceutically-active component an organogermanium compound, with or without a suitable carrier.

Organogermanium compounds have recently attracted much attention in view of their pharmacological activities. The problems with such compounds, however, are that they act differently from batch to batch, and that their specific activities remain obscure (Japanese Patent Publications Nos. 46(1971-2964 and 58-(1983)-44677).

Lately, it has been found that, like vinyl chloride and organosilicon compounds, organogermanium compounds differ slightly in their polymerizability and complex chemical structures, and exist as structures having different physical properties, resulting in the industrial preparation of their active substances having to be well established simultaneously with their identification (U.S.-A-4309412).

More recently, we have successfully developed a process for stabilizing the high activity of the analogous compounds, whereby the reproducibility of their pharmacological acti-can be confirmed. This suggests the possibility that they can be used as medicaments for various purposes (U.S. Patent No. 4889715).

The toxicity of drugs and poisonous substances to internal organs and their teratogenicity appear as side-effects in the case of the former, and present a social problem including environmental pollution in the case of the latter. These problems can never be solved without removing their underlying causes, and are thus still allowed to slumber, although the development of some antidotes is in progress.

Patients with organic diseases, especially those with liver and kidney diseases, have such impeded detoxification and excretion that, although having received drug treatment, they are likely to develop side effects such as disorders, e.g. anaphylaxis and acute organic malfunctions, or at worst to die of the treatment.

As they exhibit strong toxicity to internal organs, hard-hitting drugs for inhibiting cytotoxicity and the synthesis of nucleic acid, for example carcinostatics, are generally said to constitute double-edged swords.

The abuses of drinking and smoking, and the uptake of methyl mercury, heavy metal ions, etc. in the body cause the degeneration of tissue cells and organic disorders, which are becoming a social problem.

As already mentioned, it is believed that such problems can never be solved without removing their underlying causes. In the case of medication, the side-effect problem may be solved to some extent by reducing the dose or avoiding the use of drugs exhibiting pronounced toxicity to internal organs. For patients in need of emergent or intensive therapy, e.g. people with degenerated tissue and organs, especially those with cancer or infective diseases or pregnant women, however, it is desirable to develop the ability to use, with greater safety, drugs exhibiting a hard-hitting cytopathic action or a potentially dangerous toxic effect on internal organs. It is also desirable to establish a therapeutic procedure for preventing the impairment of tissue function associated with ageing.

Accordingly, it is an object of the present invention to provide a composition for inhibiting the degeneration of cells (including liver cells) caused by drugs, and for relieving the toxicity of drugs to internal organs including the liver.

According to the present invention, the above-mentioned object is achieved by the provision of a composition for inhibiting the degeneration of cells (including liver cells) caused by drugs and relieving the toxicity of drugs to internal organs including the liver, which contains as an essential component a 3-oxygermylpropionic acid polymer which is represented by:

$$[(O_{1/2})_3 GeCH_2 CH_2 COOH]_n$$

wherein n is an integer of at least 1, takes the form of white acicular crystal and has a specific gravity (density) of 2.23, a solubility of 1.57 in water at $20°C$ and a melting point of about $230°C$ (at which it decomposes or coagulates). The composition of this invention may contain a carrier for activating the essential component such as hydroxypropylcellulose, and may be used in combination with a substance showing an increased cytopathogenicity and/or substance showing a threatening toxicity to internal organs. The carrier may be used in an amount of 0.005 to 50% by weight relative to 0.05 to 5% by weight of the essential component.

The compositions according to this invention can be administered, with greater safety, to patients with impaired functioning of organs including the liver and the kidney, brain tissue and cells, or to pregnant women or infants with an increased proliferation of cells when they develop cancer, infective diseases or other afflictions for which they receive drug treatments, thereby relieving or eliminating the side effects of the drugs.

The compositions according to this invention can also be used to restore the functioning of cells damaged or impaired by contact with substances exhibiting strong degenerative action to tissue and a dangerous toxicity to internal organs. Moreover, the compositions according to this invention may be administered to those who are engaged in the synthesis of organic materials and so frequently run the risk of handling toxic reagents and chemicals; addicts such as habitual smokers and drinkers; or patients with chronic disease-inducing viruses, thereby preventing the development of organic diseases or their condition from becoming more serious.

Substances having a dangerous toxicity to internal organs, with which the compositions of this invention are advantageously used, include lenitives based on propionic acid such as indomethacin; antibiotics based on kanamycin; toxic substances based on mercury; alcohol; substances based on carbon tetrachloride; brain-barrier penetrating carcinostatics having a threatening toxicity to the brain; cytotoxic substances; poisonous gases based on carbon monoxide. The composition of the present invention may also advantageously be used with substances having an increased cytopathogenicity such as mercuric chloride and teratogens.

In preparing the composition of this invention, a pharmaceutical carrier may be used to make the effective component soluble or pharmacologically stable.

The composition of this invention may be used alone or in combination with drugs showing pronounced toxicity to internal organs and causing considerable tissue degeneration such as (1) antibiotics (based on penicilin, cephalosporin, kanamycin, tetracyline and streptomycin; (2) anti-phlogistics and lenitives (indomethacin); (3) carcinostatics and virocides (mitomycin, tegafur, 5-FU, cisplatin and other drugs for inhibiting cytotoxicity and the synthesis of nucleic acid); and, (4) addictive narcotics, hypnotics and tranquillizers. To this end, carriers such as lactose and albumin are preferably used to maintain the activity of the effective component. The compositions of this invention may be administered orally to patients in the form of e.g. tablets and capsules.

Although varying depending upon the type of the essential component, the type of the composition, the age of patients, etc., the composition of this invention may be administered orally to humans at a dose in the range of generally 10 to 1500 mg/kg and to adults (weighing 50 kg) at a dose of 150 mg/day.

EXAMPLES

The present invention will now be explained in greater detail with reference to the following Examples.

Preparation Example 1 - Preparation of Activated Composition

Using ethanol as a wetting agent, hydroxypropylcellulose was kneaded with 3-oxygermylpropioninic acid at 2:1, and was then dried at a temperature lower than 50°C to obtain a powdery or granular composition.

Preparation Example 2 - Tablet

The composition of Preparation Example 1 was blended with the following vehicle and other components, and the blend was then tableted in conventional manner.

| Component | Amount (mg) |
|---|---|
| Composition of Preparation Ex. 1 | 100 |
| Lactose | a suitable quantity |
| Carboxymethylcellulose (Ca) | 7 |
| Crystalline cellulose | 40 |
| Magnesium stearate | 7 |
| | 200 mg per tablet |

5

EP 0 435 693 B1

Preparation Example 3 - Tablet

The composition of Preparation Example 1 was blended with the following vehicle and other components, and the blend was then tableted in conventional manner.

| Component | Amount (mg) |
|---|---|
| Composition of Preparation Ex. 1 | 100 |
| Lactose | a proper quantity |
| Methylcellulose | 20 |
| Hydroxypropylcellulose | 8 |
| Sucrose fatty acid ester | 2 |
| Magnesium stearate | 2 |
| Sodium lauryl sulfate | 1 |
| | 200 mg per tablet |

Preparation Example 4 - Tablet

| Component | Amount (mg) |
|---|---|
| Composition of Preparation Ex. 1 | 100 |
| Indomethacin | 20 |
| Lactose | a suitable quantity |
| Methylcellulose | 20 |
| Hydroxypropylcellulose | 8 |
| Sucrose fatty acid ester | 2 |
| Magnesium stearate | 2 |
| Sodium lauryl sulfate | 1 |
| | 200 mg per tablet |

Preparation Example 5 - Capsule

A blend of the composition of Preparation Ex. 1 with the following vehicles was encapsulated in conventional manner.

| Component | Amount (mg) |
|---|---|
| Composition of Preparation Ex. 1 | 100 |
| Indomethacin | 20 |
| Lactose | 40 |
| Corn starch | 38 |
| Magnesium stearate | 2 |

Preparation Example 6 - Suppository

The composition of Preparation Ex. 1 was dispersed in a higher fatty acid glyceride, and the dispersion was then formed into a suppository in conventional manner.

6

| Component | Amount (mg) |
|---|---|
| Composition of Preparation Ex. 1 (3-oxygermylpropionic acid) | 60 (20) |
| 5-fluorouracil | 100 |
| Cacao butter | 1540 |
| | 1700 mg per suppository |

Pharmacological Test Example I

I-A. Toxicity Reagent

With (a) the 3-oxygermylpropionic acid according to this invention (hereinafter called SK-818) and (b) the composition of Preparation Ex. 1, SD rats and mice were subjected to acute and subacute (chronic) toxicity tests in conventional manner. The results are reported below. (Groups of animals, 8-10 per group, were used for experimentation).

i. Acute Toxicity

| LD50 (mg/kg) | | per os (no significant difference between (a) and (b)) |
|---|---|---|
| Mice, | male | 5,600 or more |
| | female | 5,800 or more |
| Rats, | male | 7,700 or more |
| | female | 7,050 or more |

Between (a) SK-818 and (b) the composition of Preparation Ex. 1 there was no significant difference in acute toxicity. While the test group to which SK-818 was administered developed general symptoms of tranquilization, diarrhoea, vomiting and typhlectasis, the test group to which received the composition of Preparation Ex.1 did not substantially show any special symptoms.

ii. Subacute Toxicity

(a) SK-818 and (b) the composition of Preparation Ex. 1 were administered orally to SD masculine rats at doses of 256, 380, 640, 1300, 1600 and 4000 mg/kg/day over three months to determine the doses at which there was no toxic influence on the animals and the animals were absolutely poisoned, respectively.

The dose of SK-818 which had no toxic influence on the animals was 256 mg/kg, and the dose of SK-818 at which the animals were absolutely poisoned was 1600 mg/kg. At 4000 mg/kg, some animals died. After the lapse of five weeks, however, the survivors recovered from poisoning.

The dose of the composition of Preparation Ex. 1 which had no toxic influence on the animals was 380 mg/kg, and its dose causing the animals to be absolutely poisoned was 1300 mg/kg. All the animals were left alive.

I-B. Effects on inhibiting the heteroplasia of liver cells and relieving hepatoxicity in liverish models

Effect on relieving hepatoxicity in rats with hypohepatia

Because the composition of Preparation Ex. 1 was found to be more efficacious than SK-818 in the subacute toxicity test, the following experimentation was carried out with this composition.

Carbon tetrachloride was administered orally as a hepatoxic substance to SD masculine rats (weighing 500 g ± 50 g; age, 10 months) with hypohepatia (3 groups containing 8-9 rats each) at a dose of 0.5 g/kg, whereby their serum GOT was increased to more than 50 Karmen units after 24 hours. At the same time, 2 mg/kg of indomethacin as a hepatoxic drug (a lenitive), 5 mg/kg of 5-FU as a hepatoxic carcinostatic and 10 mg/kg of tetracycline as a hepatoxic antibiotic were administered orally to each of the animals five times every fourth day.

An additional 20 mg/kg of the composition of Preparation Ex. 1 was administered orally to the medicated group.

Seven days after the completion of medication, the animals were slaughtered to remove the livers, which were then visually observed. Afterwards, the tissue slices were observed under an optical microscope to examine the degree of heteroplasia of the liver cells.

The GOT eluates due to cytoclasis were determined by gathering blood from the tail vein of each animal 24 hours after the administration of carbon tetrachloride, two and ten days after the initiation of medication and on the final date of experimentation.

From the results reported in Table 1, it was found that, in the test groups, blood-GOT levels begun increasing just after the administration of the hepatoxic substances and reached more than 80 Karmen units signifying the aggravation of hepatopathy while, in the groups treated with the composition of Preparation Ex. 1, there was a significant drop of blood-GOT levels.

Referring to the degree of heteroplasis of liver cells examined by autopsy, the assay of tissue indicated that even in the control group, there were corpulence due to the aged animals and a decrease in the number of basophiles over the lobule. In the groups to which only the hepatoxic substances were administered a number of fibrogeneses were found and a part of the liver tissue contained a cellular colony containing such cells as large and polynuclear cells. Even in the groups to which indomethacin and 5-FU were administered some animals died. In the test groups which were further treated with the composition of Preparation Ex. 1, all the animals were left alive. The results of autopsy indicated that there were corpulence and some tissue necroses, but the assay of tissue showed that there was no heteroplasis of liver cells.

## Table 1

### Changes in serum GOT in Karmen units/ml

$51.3\pm2.31$ as measured 24 hours after the administration of carbon tetrachloride

|  | 2 days after the initiation of medication | 10 days | Final Date |
|---|---|---|---|
| Control group | $64.0\pm5.47$ | $25.6\pm3.54$ | $23.8\pm1.96$ |
| Test groups |  |  |  |
| (i) | $81.3\pm4.21$ | $91.8\pm8.35$ | $99.4\pm1.43$ |
| (ii) | $78.5\pm2.31$ | $83.8\pm2.41$ | $96.1\pm3.24$ |
| (iii) | $83.2\pm6.35$ | $82.0\pm5.97$ | $89.6\pm2.51$ |
| Test groups treated with the composition of Preparation Ex.1 |  |  |  |
| (i) | $71.3\pm2.31$ | $46.4\pm1.35$ | $15.3\pm2.53$ |
| (ii) | $73.6\pm6.35$ | $43.2\pm3.54$ | $19.3\pm3.18$ |
| (iii) | $80.1\pm3.72$ | $56.1\pm2.71$ | $12.0\pm8.31$ |

(i): Indomethacin

(ii): 5-FU

(III): Tetracycline

I-C. Effect on inhibiting the action of a hepatocyte-damaging substance on cultivated liver cells

The effect on relieving the culture damage induced by adding hepatocyte-damaging substances and SK-818 to hepatocyte culture systems was examined by measuring the activity of glutamic-pyruvic transaminase (GPT) eluates obtained by cellular disorders. How much the elution of GPT was inhibited was used as an index to the hepatoxicity-relieving effect.

The following four substances were used as the hepatocyte-damaging (hepatoxic) substances.
(a) Galactosamine - "J. Natur. Prod.", 46, 841, 1983.
(b) Calcium ionophore - "Pharmacognosy", 39, 218, 1985.
(c) Cumene hydroperoxide - "Planta Med.", 51, 50, 1985.
(d) 1-Naphthylisothiocyanate.

The extraction and culture of a single layer of rat's hepatocytes were carried out by the procedures described by Nakamura et al in "Protein, Nucleic Acid and Oxygen", Extra Number No. 24, pp. 55-74, 1981.

Single layers of rats' hepatocytes were cultured for certain periods of time:- 0 hour for a galactosamine group, 20.5 hours for calcium ionophore and cumene hydroperoxide groups, and 14.5 hours for a 1-naph isocyanate group. Afterwards, the hepatocyte-damaging substances were added to the culture media at the respective desired concentrations:- 0.35 mM for (a); 10 $\mu$m for (b), 0.5 mM for (c) and 188 $\mu$m for (d)), which were then cultured for the respective given periods of time:- 38 hours for the group (a), 1 hour for (b), 1 hour for (c) and 12 hours for (d).

SK-818 was dissolved in bovine embryo serum, and the serum solution was then added to each medium at the respective stages of culture by the following three methods;
(a) it was added to the pre-culture medium prior to the addition of the hepatocyte-damaging substance (called the pre-addition);
(b) it was added to the culture medium simultaneously with the addition of the hepatocyte-damaging substance (called the simultaneous addition); and
(c) it was added to the pre-culture and culture media (called the total addition).

The activity of the GPT eluates produced by cytotoxicity in the media was measured by a GPT measuring kit. Then, GPT reductions in % were found by the following equation:

$$\text{GPT reductions (\%)} = \frac{(B-A) - (C-A)}{(B-A)} \times 100$$

wherein

A is the activity in Karmen units of GPT in a culture medium to which no hepatocyte-damaging substance is added,

B is the activity in Karmen units of GPT in a culture medium to which the hepatocyte-damaging substance is added; and

C is the activity in Karmen units of GPT in a culture medium to which the hepatocyte-damaging substance is added together with 3-oxygermylpropionic acid.

Results

The following GPT reductions were obtained.
(a) Addition of galactosamine
29 % in the case of the simultaneous addition of SK-818.
(b) Addition of calcium ionophore
27 % in the case of the simultaneous addition of SK-818, and
36 % in the case of the total addition of SK-818.
(c) Addition of cumene hydroperoxide
63 % in the case of the simultaneous addition of SK-818, and
42 % in the case of the total addition of SK-818.
(d) Addition of 1-naphthylisothiocynate
79 % in the case of the simultaneous addition of SK-818, and

67 % in the case of the total addition of SK-818.

No GPT reduction was achieved only by the pre-addition of SK-818. In other words, it was only when SK-818 was used in combination with the hepatocyte-damaging substances that significant GPT reductions were obtained. This implies that SK-818 has an extracellular inhibiting effect.

I-D. Clinical test of SK-818 for Type B chronic hepatitis

(a) Object

The efficacy of SK-818 against Type B chronic hepatitis was examined, using a placebo as control.

(b) Subject

The subjects were patients who had been diagnosed by biopsy as chronic hepatitis before at most one year in principle, were positive to HBe antigens and had Type B chronic hepatitis in need of treatment.

(c) Drug under test and control

(i) The drug under test was a capsule containing 10 mg of SK-818.
(ii) The control consisted only of lactose (a placebo).

(d) Administration

The subjects followed the standard prescription of three capsules a day and one capsule each meal over 16 weeks in principle.

(e) What was measured:

The general examination of the functioning of the livers and blood and the measurement of HBe antigens and antibodies were all carried out by the Virus Hepatitis Research Foundation.

(f) Participants

Table 2

| | | Number of cases | | |
|---|---|---|---|---|
| | | SK-818 | Placebo | Total |
| Sex | male | 81 | 63 | 144 |
| | female | 20 | 18 | 38 |
| Age | ~19 | 2 | 1 | 3 |
| | 20~29 | 31 | 23 | 54 |
| | 30~39 | 39 | 27 | 66 |
| | 40~49 | 18 | 21 | 39 |
| | 50~59 | 9 | 5 | 14 |
| | 60~ | 2 | 4 | 6 |
| | Average | 35.2 | 37.1 | 36.0 |
| Duration | CAH | 78 | 63 | 141 |
| | CIH | 17 | 13 | 30 |
| Day-patients In-patients | Day-patients | 83 | 73 | 156 |
| | In-patients | 4 | 1 | 5 |
| | Day-and In-patients | 14 | 7 | 21 |
| Complications | not developed | 89 | 72 | 161 |
| | developed | 12 | 9 | 21 |

(g) Results

Diagnoses of the physicians in charge as to how the functioning of the livers was ameliorated are reported in Table 3 and the statistics in Table 4.

As will be seen from the rate of amelioration of "slightly ameliorated - 57. 9 %" or more reported in table 4, most of the patients show a reduced or limited elution of enzymes in liver cells. This suggests that the activity of Type B hepatitis-associated viruses is-inhibited by SK-818.

Table 3

Degree of Amelioration (determined by physicians)

| Estimation / Drugs | Remarkably ameliorated | Ameliorated to some extent | Slightly ameliorated | Nochange | Less aggravated | aggravated | seriously aggravated | Total | Rate of Amelioration | |
|---|---|---|---|---|---|---|---|---|---|---|
| | | | | | | | | | Ameliorated to some or higher extent | Slightly ameliorated or more |
| SK−818 | 12 | 23 | 20 | 31 | 5 | 4 | 0 | 95 | 36.8 | 57.9 |
| Placebo | 6 | 6 | 9 | 22 | 16 | 8 | 1 | 68 | 17.6 | 30.9 |
| Total | 18 | 29 | 29 | 53 | 21 | 12 | 1 | 163 | | |
| Mann-Whitney's U Assay | $Z_0 = 3.997$   $P = 0.0001$*** | | | | | | | Fisher's direct calculation | P=0.0114* | P=0.0011** |

\*:P<0.05  \*\*:P<0.01  \*\*\*:P<0.001

( P : accuracy(probability) )

EP 0 435 693 B1

Table 4
Statistics

| | SK-818 | | | | | | Placebo | | | | | | Differences between groups |
|---|---|---|---|---|---|---|---|---|---|---|---|---|---|
| | 4W | 8W | 12W | 16W | Af4W | Af8W | 4W | 8W | 12W | 16W | Af4W | Af8W | |
| HBe antigens (EIA) or | ↓*** | ↓** | ↓*** | ↓*** | ↓*** | ↓*** | | | | | | ↓* | 4W** 12W* / 16W** Af4w* |
| HBe antibodies (RIA%) | ↑* | ↑* | ↑** | ↑* | ↑* | ↑** | | | | | | | 4W* 12W* / 16W*‡ Af8w+ |
| GOT | ↓* | ↓* | ↓*** | ↓*** | ↓*** | ↓** | | | | | | | 12W+ 16W+ |
| GPT | ↓* | ↓** | ↓*** | ↓*** | ↓*** | ↓** | | | | | | | 8w* 12w*** / 16W* Af4w* |
| LDH (nu/ml) | ↑+ | ↓+ | ↑+ | | | | | | | | | | |
| AlP (KA) | ↓+ | ↓+ | ↓+ | | | | | | | | | | |
| r-GTP (nu/ml) | | | ↓** | ↓* | ↓** | ↓* | | | | | | | 16w+ |
| Total Bilirubin (mg/dl) | ↓* | | | | | | | | | | | | 4w+ 8W* |
| Direct Bilirubin (mg/dl) | | | | | | | | ↑** | | | | | 4w+ 8W* |
| Cholinesterase (ΔPH) | | ↑* | ↑* | ↑* | ↑* | ↑** | ↑* | ↑+ | ↓+ | | | | 4w* 12W** Af4W* / 8w*** 16w* Af8w+ |
| Total serum Protein (g/dl) | | | | | | | | | | | | | |
| Total Cholesterol (mg/dl) | | ↑* | ↑* | ↑* | ↑* | ↑* | | | | | | | 8W* 16W** / 12W** Af8w* |
| Serum Albumin (g/dl) | | | ↑* | ↑+ | | | | | ↑+ | | | | Af4w+ |
| TTT | | ↑* | ↓** | ↓*** | ↓*** | ↓* | ↓+ | ↓* | ↓+ | ↓+ | | | |
| ZTT | | ↓* | ↓** | ↓** | ↓** | ↓* | | ↓** | ↓* | | | | |
| r-Globulin (g/dl) | | | ↓** | ↓*** | ↓*** | ↓* | ↓+ | ↓** | ↓* | | | | 12W+ Af4w* / 16w+ Af8w+ |

表中 ***:P<0.001 **:P<0.01 *:P<0.05 +:P<0.10 ↑:rise (increase)
Af4W:after 4 weeks Af8W:after 8 weeks ↓:drop(decrease)
W:Weeks

Pharmacological Test Example II

II-A. Action of SK-818 on preventing nephritis

(a) Object

The action of SK-818 on preventing the development of spontaneous nephritis of MRL/1 mice (or an impairment of the functioning of the kidney asaociated with aging) was examined.

(b) Procedures

Given amounts (0.1 mg/kg, 1.0 mg/kg and 10.0 mg/kg a day) of SK-818, in the form of a mixture of the composition of Preparation Ex. 1 with feed, were administered orally MRL/1 masculine mice of six-week age (16 for each group) twice a week over 12 weeks. In the meantime, urinary protein was measured once a week; urinary protein levels exceeding 100 mg/dl were taken as positive. Examination was then made of how many animals became positive at the respective week-ages. Moreover, blood-urinary nitrogen levels

13

were determined on the day following the final administration to make serological/physiological and pathological estimations of the action of SK-818 on nephritis.

The urinary protein and blood-urinary nitrogen levels were measured with Protein Pre-test (made by Wako Junyaku K.K.) and Urinary Nitrogen B-Test according to the urease/indophenol method (made by Wako Junyaku K.K.), respectively.

(C) Results

The results are reported in Tables 5 and 6.

As can be seen from the data, the control group shows an increase in the urinary protein levels from 15 or 16 week-age, but the test groups do not. At 10 mg/kg, the increase in the urinary protein levels is remarkably inhibited (see Table 5).

This also holds for the blood-urinary nitrogen levels. At 10 mg/kg, the increase in the blood-urinary nitrogen levels is significantly inhibited (see Table 6).

In general, MRL/1 mice develop major symptoms of membranoproliferative glomerulonephritis. In the control group, 57 % of glomeruli becomes more morbid, but only 23-34 % does in the groups medicated with SK-818. Thus, it is found that SK-818 produces an inhibitory action on the spontaneous development of nephritis in MRL/1 mice.

Altogether, SK-818 is efficacious against the development of spontaneous nephritis in MRL/1 mice from the standpoints of serology/physiology and pathology.

Table 5

| Number of animals with positive urinary protein levels | | | |
|---|---|---|---|
| Amount of SK-818 | 16 week-age | 17 week-age | 18 week-age |
| Control | 7/15 | 9/15 | 13/15 |
| 0.1 mg/kg | 4/15 | 7/15 | 10/15 |
| 1 mg/kg | 4/15 | 6/15 | 8/15 |
| 10 mg/kg | 2/15 | 4/15 | 8/15 |

In Table 5, 7/15, for instance, means that 7 in 15 became positive.

Table 6

| Blood-urinary nitrogen levels | |
|---|---|
| Amount of SK-818 | BUN (mg/kg) |
| Control | 39.5±3.9 |
| 0.1 mg/kg | 32.2±1.6 |
| 1 mg/kg | 35.5±3.3 |
| 10 mg/kg | 29.3±1.7 |

II-B. Action of SK-818 on relieving the nephrotoxicity of mercuric chloride

(a) Object

The effect of SK-818 on the impairment of kidney-function by mercuric chloride was examined, using blood-urinary nitrogen levels as an index.

(b) Procedures

Mercuric chloride was subcutaneously administered to Wistar masculine rats (seven weeks of age) 6-10 per group, at a dose of 1.2 mg/kg. From 24 hours after the administration till the lapse of 96 hours, the animals were permitted to freely drink an aqueous solution containing 2,400 ppm of SK-818 (at an average dose of 231±14 mg/kg). Blood was gathered before and just after the administration of mercuric chloride as well as the lapse of 24, 48, 72 and 96 hours to measure blood-urinary nitrogen levels.

(c) Results

The results are reported in Table 7.

The groups all showed a maximum blood-urinary nitrogen level after 48 hours. In the group medicated with SK-818, however, the blood-urinary nitrogen levels decreased sharply from the peak value, as time went by. This suggests that SK-818 is efficacious against impairment of the functioning of the kidneys caused by the administration of mercuric chloride.

Table 7
(Effect of SK-818 on blood-urinary nitrogen levels after administration of mercuric chloride)

| Groups | Blood-urinary nitrogen (mg/dl) | | | | |
|---|---|---|---|---|---|
| | 0 hour | 24 hours | 48 hours | 72 hours | 92 hours |
| Untreated (n=6) | 19.1±0.3 | — | 15.8±0.6 | — | 14.1±0.5 |
| Control (n=10) | 18.0±0.4 | 36.1±1.9 | 69.5±11.1 | 64.8±19.6 | 43.0±17.4 |
| SK-818 (2400ppm aqueous solution) | 15.1±0.4 | 31.9±1.3 | 50.6±9.8 | 45.9±13.8 | 30.9±9.4 |

II-C. Effect of SK-818 on amnesia induced by carbon dioxide

(a) Procedures

A ddy masculine mouse was encaged in a dark chamber of a testing box (comprising bright and dark chambers, each measuring 15.0 x 17.5 x 18.5 cm and provided with an inlet/outlet combination of 6.0 x 6.0 cm, and the time which it took the animal to walk into the dark chamber (the reaction potential time during acquisition, hereinafter referred to as A.T.) was then measured. From just after the animal walked into the dark chamber, foot shocks of 2.5 mA were continuously applied to the animal through a floor grid with a shock generator scrambler, made by Astech Co., Ltd., until the animal walked into the bright chamber. Immediately after the acquisition trial, the animal was encaged in a desiccator, and 15 l/min of $CO_2$ gas were injected into the desiccator for 40 to 45 seconds for suffocation. After the animal was removed from the desiccator, artificial aspiration was immediately tried thereon. The animal was then put back in a home cage.

After 24 hours of the acquisition trial, the animal was again encaged in the bright chamber of the testing box to measure the time which it took the animal to walk into the dark chamber (or the reaction potential time during the retention trial, hereinafter referred to as R.T.). SK-818, in the form of a mixture with feed, had been administered orally to the animal from 3 days before at a dose of 10.0 mg/kg/day.

(b) Results

The results of dysmnesia models induced by the of $CO_2$ gas are reported in Table 8, from which it is found that SK-818 gives rise to a significant increase in R.T., and so is efficacious against dysmnesia.

Table 8

| | | Number of animals | A.T. In sec. | R.T. in sec. |
|---|---|---|---|---|
| Group | A | | 51.8±9.1 | 189.4±19.2 |
| | B | 26 | 48.5±9.1 | 276.5±19.3 |
| | C | 15 | 50.7±19.7 | 360.0 or longer |
| | C | 15 | 51.3±5.2 | 68.3±11.1 |
| Group | | | | |
| A: Foot Shocks + $CO_2$ Gas | | | | |
| B: SK-818 + Foot Shocks + $CO_2$ Gas | | | | |
| C: Foot Shocks | | | | |
| D: $CO_2$ Gas | | | | |

**Claims**

1. Use of a composition for the manufacture of a medicament for inhibiting the degeneration of liver cells caused by drugs and for relieving the toxicity of drugs to the liver or kidneys, characterised in that the composition contains as an essential component a 3-oxygermylpropionic acid polymer which is represented by the following formula:

$$[(O_{1/2})_3 GeCH_2 CH_2 COOH]_n$$

wherein n is an integer of at least 1, said 3-oxygermylpropionic acid polymer being in the form of white acicular crystals and having a melting point of about 230°C at which temperature it decomposes or coagulates.

2. Use as claimed in Claim 1 wherein the composition additionally comprises a carrier for activating said essential component.

3. Use as claimed in Claim 2, wherein said carrier is hydroxypropylcellulose present in an amount of 0.005 to 50% by weight relative to 0.005 to 5% by weight of said 3-oxygermylpropionic acid polymer.

4. Use as claimed in Claim 1, 2 or 3 against a substance exhibiting pronounced cytopathogenicity.

5. Use as claimed in Claim 1, 2 or 3, against a substance exhibiting potentially dangerous toxicity to the liver and kidneys.

6. Use as claimed in Claim 4, wherein said substance showing an increased cytopathogenicity is mercuric chloride or a teratogen.

7. Use as claimed in Claim 5, wherein said substance exhibiting potentially dangerous toxicity exhibits toxicity to the kidneys and is a propionic acid type lenitive, an antiobiotic or a mercury type toxic substance.

8. Use as claimed in Claim 5, wherein said substance exhibits potentially dangerous toxicity exhibits toxicity to the kidneys and is a brain-barrier penetrating carcinostatic, a cytotoxic substance or a carbon tetrachloride type poisonous gas.

9. Use as claimed in Claim 1, 2 or 3, wherein said composition is used in combination with a drug, substance or compound having a pronounced hepatoxicity.

10. Use as claimed in Claim 9, wherein said drug is an indomethacin type lenitive, a streptomycin, kanamycin, cephalosporin, penicillin or mitomycin type antibiotic, or a 5-FU or platinum complex type carcino-static; said compound is based on an organic, inorganic or biological system; and said substance is an alcoholic or carbon tetrachloride type substance.

**Patentansprüche**

1. Verwendung einer Zubereitung zur Herstellung eines Medikaments zur Hemmung der durch Drogen verursachten Leberzellendegeneration und zur Linderung der Toxizität von Drogen bezüglich der Leber oder der Nieren, dadurch gekennzeichnet, daß die Zubereitung als eine wesentliche Komponente ein 3-Oxygermylpropionsäurepolymer enthält, das durch die folgende Formel dargestellt ist:

$[(O_{1/2})_3 GeCH_2 CH_2 COOH]_n$,

worin n eine Zahl von wenigstens 1 ist, wobei das 3-Oxygermylpropionsäurepolymer in Form weißer Kristallnädelchen vorliegt und einen Schmelzpunkt von ungefähr 230°C besitzt, bei dem er sich zersetzt oder koaguliert.

2. Verwendung nach Anspruch 1, worin die Zubereitung zusätzlich einen Träger zur Aktivierung der wesentlichen Komponente beihaltet.

3. Verwendung nach Anspruch 2, worin der Träger Hydroxypropylzellulose ist und in einer Menge von 0,005 bis 50 Gew.-% im Verhältnis zu 0,005 bis 5 Gew.-% des 3-Oxygermylpropionsäurpolymers vorliegt.

4. Verwendung nach Anspruch 1, 2 oder 3, gegen eine Substanz, die eine ausgeprägte Cytopathogenität aufweist.

5. Verwendung nach Anspruch 1, 2 oder 3, gegen eine Substanz, die eine potentielle gefährliche Toxizität bezüglich der Leber und der Nieren aufweist.

6. Verwendung nach Anspruch 4, worin die Substanz, die eine erhöhte Cytopathogenität aufweist, Quecksilberchlorid oder ein Teratogen ist.

7. Verwendung nach Anspruch 5, worin die die potentielle gefährliche Toxizität aufweisende Substanz eine Toxizität bezüglich der Nieren aufweist und ein Linderungsmittel vom Propionsäure-Typ, ein

EP 0 435 693 B1

Antibiotikum oder eine toxische Substanz vom Quecksilber-Typ ist.

8. Verwendung nach Anspruch 5, worin die die potentielle gefährliche Toxizität aufweisende Substanz eine Toxizität bezüglich der Nieren aufweist und ein die Blut-Hirn-Schranke überwindendes krebshemmendes Mittel, eine cytotoxische Substanz oder ein giftiges Gas vom Kohlenstofftetrachlorid-Typ ist.

9. Verwendung nach Anspruch 1, 2 oder 3, worin die Zubereitung in Kombination mit einer Drogensubstanz oder einer Verbindung mit einer ausgeprägten Lebertoxizität verwendet wird.

10. Verwendung nach Anspruch 9, worin die Droge ein Linderungsmittel vom Indomethazin-Typ, ein Antibiotikum vom Streptomyzin-, Kanamyzin-, Cephalosporin-, Penicillin- oder Mitomyzin-Typ, oder ein krebshemmendes Mittel vom 5-FU- oder Platinkomplex-Typ ist; wobei die Verbindung auf einem organischen, anorganischen oder biologischen System basiert; und wobei die Substanz eine alkoholische Substanz oder eine Substanz vom Kohlenstofftetrachlorid-Typ ist.

## Revendications

1. Utilisation d'une composition pour la fabrication d'un médicament destiné à inhiber la dégénérescence de cellules hépatiques sous l'effet de médicaments et à diminuer la toxicité de médicaments à l'égard du foie ou des reins, utilisation caractérisée en ce que la composition contient, à titre de constituant essentiel, un polymère de l'acide 3-oxygermylpropionique, qui est représenté par la formule suivante :

$$[(O_{1/2})_3 GeCH_2 CH_2 COOH]_n$$

(dans laquelle n est un nombre entier valant au moins 1), ledit polymère d'acide 3-oxygermylpropionique étant sous forme de cristaux aciculaires blancs présentant un point de fusion d'environ 230°C, température à laquelle le composé se décompose ou coagule.

2. Utilisation selon la revendication 1, dans laquelle la composition comprend en outre un véhicule ou excipient pour activer ledit constituant essentiel.

3. Utilisation selon la revendication 2 dans laquelle ledit excipient est de l'hydroxypropyl cellulose présente en une quantité de 0,005 à 50 % en poids par rapport à 0,005 à 5 % en poids dudit polymère d'acide 3-oxygermylpropionique.

4. Utilisation selon la revendication 1, 2 ou 3 contre une substance exhibant un caractère cytopathogène prononcé.

5. Utilisation selon la revendicaiton 1, 2 ou 3, contre une substance présentant une toxicité potentiellement dangereuse pour le foie et les reins.

6. Utilisation selon la revendication 4, dans laquelle ladite substance présentant un caractère cytopathogène accru est le chlorure mercurique ou une substance tératogène.

7. Utilisation selon la revendication 5, dans laquelle ladite substance présentant une toxicité potentiellement dangereuse présente de la toxicité pour les reins et est un lénitif du type acide propionique, un antibiotique ou une substance toxique du type comportant du mercure.

8. Utilisation selon la revendication 5 dans laquelle ladite substance présentant un caractère potentiellement dangereux de toxicité présente de la toxicité pour les reins et est une matière carcinostatique pouvant traverser la barrière hémato-encéphalique, une substance cytotoxique ou un gaz toxique du type tétrachlorure de carbone.

9. Utilisation selon la revendication 1, 2 ou 3, dans laquelle ladite composition sert en combinaison ou association avec un médicament ou une drogue, une substance ou un composé ayant un caractère prononcé d'hépatotoxicité.

18

**10.** Utilisation selon la revendication 9, dans laquelle ledit médicament est un lénitif du type indométhacine, un antibiotique du type streptomycine, kanamycine, céphalosporine, pénicilline ou mitomycine, ou un carcinostatique de type 5-FU ou complexe de platine; ledit composé est à base d'un système organique, minéral ou biologique; et ladite substance est une substance alcoolique du type tétrachlorure de carbone.